# EUROPEAN PATENT SPECIFICATION

(11) **EP 1 165 163 B1**
(45) Date of publication and mention of the grant of the patent: **09.06.2004**
(21) Application number: 00917267.7
(22) Date of filing: 29.03.2000
(51) Int. Cl.: A61M 5/50

(54) **SINGLE-USE SYRINGE CONSTRUCTED TO PREVENT ITS SUBSEQUENT RE-USE**
EINWEGSPRITZE MIT EINRICHTUNG ZUR VERHINDERUNG MEHRMALIGEN GEBRAUCHS
SERINGUE A USAGE UNIQUE CONçUE POUR EMPECHER SA REUTILISATION

(30) Priority: 02.04.1999 IT FI990076
(43) Date of publication of application: 02.01.2002
(73) Proprietor: Masi, Mauro, 53100 Siena (IT)
(72) Inventor: Masi, Mauro, 53100 Siena (IT)
(74) Representative: Mannucci, Michele
(86) International application number: PCT/IT2000/000107
(87) International publication number: WO 2000/059564

(56) References cited:
- FR-A- 2 653 340
- US-A- 5 078 686
- US-A- 5 149 323
- US-A- 5 201 709
- US-A- 5 226 884

## Description

The repeated use of a syringe of the single-use type frequently occurs; this can cause serious harm because of possible infection and contagion. However, any solution for preventing re-use of a single-use syringe must be not only safe but also very simple and must be provided at a low and affordable cost, since the single-use syringe must be offered at a low price because of the limited service which it provides.

The invention provides a complete solution to this problem and also permits, if desired, a practically total use of the equipment and components used hitherto for the production of components of single-use syringes of the standard type.

These and other objects and advantages will be made clear by the following text.

The single-use syringe comprises a barrel with a needle, a piston made from elastomeric material, and a piston rod; in addition to these elements, said syringe comprises - to prevent its subsequent re-use according to the invention - a disengageable connection between the piston and the rod, and also a component which is capable of establishing the said connection and which is in frictional contact with the inner surface of the barrel. Said component is thus capable of being moved by the piston when the latter is made to slide in the direction of suction, and maintains the connection between the piston and the rod in these conditions; the disengagement of the piston from the rod is caused by the reverse stroke, in other words by the injection stroke, which the piston is made to carry out by the rod, while said tubular component continues to be retained by friction on said inner surface of the barrel and thus loses the capacity to maintain the connection between the rod and piston.

Said disengageable connection of the expansion type is provided by a mushroom head of the piston rod and a socket created in an extension of the piston which is capable of expanding as a result of cuts at the position of said socket; while said extension remains surrounded by said tubular component, said expansion is prevented and thus the disengagement of the rod and piston is prevented.

Said extension is formed by an intermediate element provided with an appendage shaped in the same way as the mushroom head of the piston rod: said appendage can thus be engaged in the socket which is normally provided in the piston to receive the mushroom head of the rod, so that the appendage replaces the latter mushroom head.

More generally, the improved single-use syringe according to the invention comprises: a disengageable connection between the piston and rod; a component capable of establishing said connection with flexible parts is in frictional contact with the inner surface of the barrel and can be moved by the piston when the latter is made to slide in the suction direction and, in these conditions, can maintain the connection between the piston and rod. The disengagement of the piston from the rod is caused by the reverse stroke for injection, which the piston is made to carry out by a thrust from the rod, while said component is retained by friction on said inner surface of the barrel. This disengagement can be caused by an inward (rather than an outward) deformation of the flexible parts, which can be formed on the friction-creating component, while the tubular containing wall can be formed by the piston.

A syringe comprising the features of the preamble of claim 1 is disclosed in document US 5,149,323.

The invention will be more clearly understood from the description and the attached drawing, which shows a practical, non-restrictive example of the invention. In the drawing:
Fig. 1 shows a syringe in longitudinal section, ready to start the operations of suction and subsequent injection;
Fig. 2 shows the syringe in the configuration in which the suction of the liquid has taken place, with the presence of possible air bubbles in conditions of expulsion of the air;
Fig. 3 shows the stage of penetration of the needle into the connective tissue;
Fig. 4 shows the stage of checking the correct position of the needle, when an intramuscular injection is to be carried out;
Fig. 5 shows the end of the stage of injection into the cutaneous tissue and removal of the needle from the cutaneous tissue;
Fig. 6 shows the configuration in which the rod is disengaged from the piston, and which the syringe assumes if an attempt is made to carry out a subsequent suction followed by a subsequent use;
Figs. 7, 8 and 9 show in detail one of the components of the arrangement shown in the preceding figures; and
Fig. 10 shows a variant embodiment.

The drawing shows an embodiment of the invention in which a known arrangement of a single-use syringe is used. The number 1 indicates the needle, and 3 indicates the core in which the needle is fitted and which, in turn, can be engaged with the barrel 5 of the syringe or made in one piece with the barrel 5. The number 6 indicates the normally used piston of such a syringe, which can be made from a relatively soft material such as synthetic rubber or other. The number 7 indicates the piston operating rod, which is usually engaged with the piston by means of a mushroom head 7A which is made to penetrate into a socket 6A formed in the piston 6; this penetration of the mushroom head 7A into the socket 6A can be carried out before the insertion of the piston 6 into the barrel 5 of the syringe, or by making use of the elastic yield of the material of the piston 6 (when this is sufficiently elastic), while the piston 6 is already housed in the barrel 5 of the syringe.

A syringe made in this way can be re-used after a first use for suction and injection, since the rod 7 of the piston 6 can continue to operate the piston, making it slide along the inner surface 5A of the wall of the barrel 5 of the syringe. The invention can be used to prevent this possibility of use after the first use of the syringe, which must be of the disposable type to avoid all the problems of contamination and infection which a repeated use of a needle of a disposable syringe can cause.

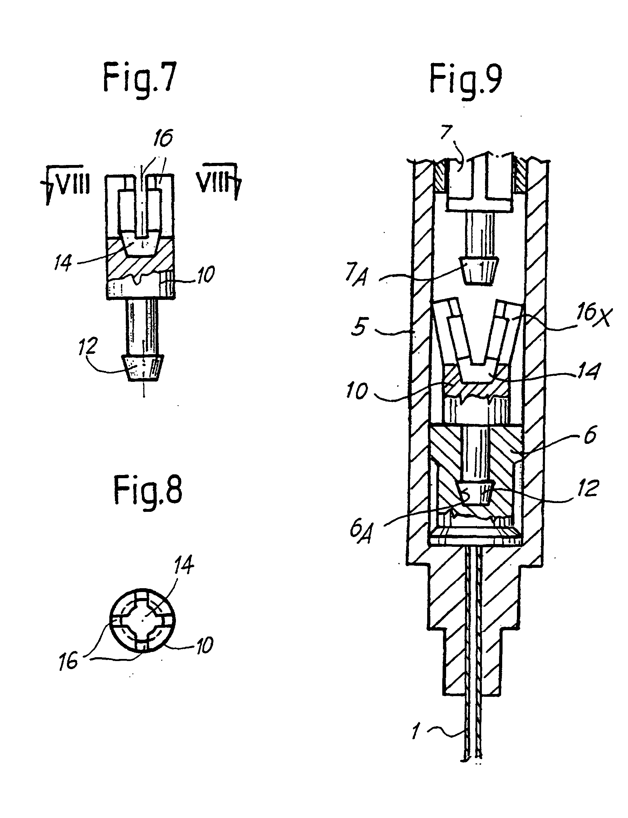

According to the solution illustrated in the drawing, the components 6 and 7 of the known conventional syringe are still used . An intermediate element 10 is used; this extends essentially in the form of a small cylinder which has an appendage 12 essentially identical to the mushroom head 7A of the rod 7, and which has at its opposite end a housing 14 essentially corresponding to the socket 6A of the piston 6, with the difference that the end of the element 10 forming the socket 14 is divided by two longitudinal cross cuts 16, in such a way that said end can spread, in other words expand as indicated in Fig. 9, the element 10 being of a sufficiently elastic material to allow this deformation.

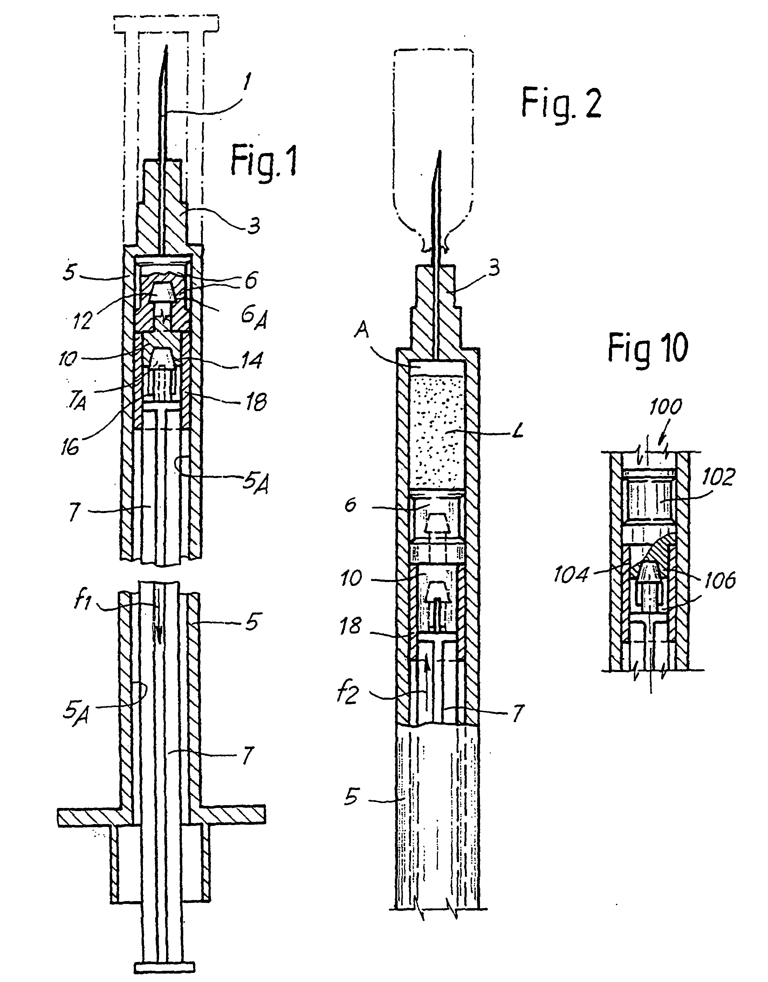

A tubular component 18, capable of sliding inside the barrel 5 with a degree of friction with the inner surface 5A of said barrel 5, is also used; the tubular component 18 can surround the intermediate element 10, in the conditions shown in Figs. 1 to 4. The intermediate element 10 is engaged with the piston 6 and the tubular component 18 is fitted on this element, and the assembly is inserted into the barrel 5; the rod 7 is then fitted, so that the mushroom-shaped appendage 7A of the rod is engaged in the housing 14 after the insertion of said assembly into the barrel.

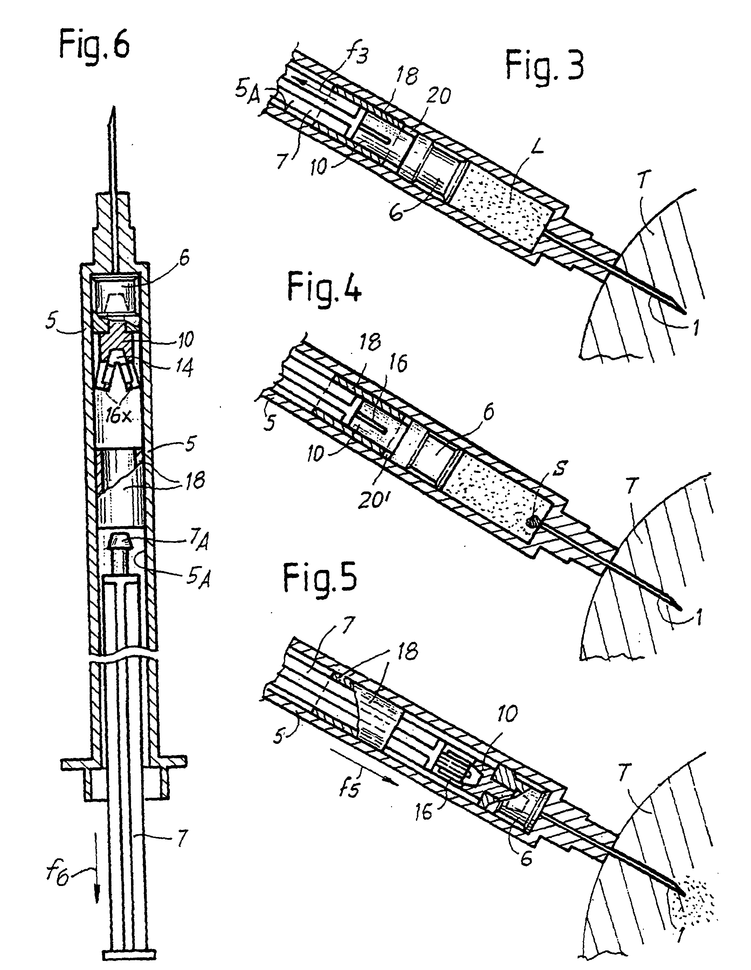

When the assembly is in the conditions shown in Fig. 1, the mushroom head 7A remains engaged by the intermediate element 10 with the piston 6, by means of the appendage 12 engaged in the socket 6A, the tubular component 18 being in the condition in which it prevents the elastic spreading of the portions of the element 10 which are delimited by the cuts 16. In this condition, the piston 6 can be operated by the rod 7 so that it is made to slide as shown by the arrow f1 in the conditions of suction of the liquid. This sliding also moves the tubular component 18, which bears against the end of the piston 6 opposite that used for the suction and the injection. The liquid L is therefore sucked up by operation in the direction of the arrow f1 in Fig. 1; any air A which may be sucked up with the liquid L, as seen in Fig. 2, can be expelled by a brief movement of the rod 7 in the reverse direction to that of the arrow f1, and the consequent movement of the piston 6 in the direction of the arrow f2 in Fig. 2. When the air has been expelled, in the conditions shown in Fig. 3 the needle 1 can be made to penetrate into the tissue T in which the intramuscular injection is to be carried out, or into a vein for an intravenous injection. When an intramuscular injection is to be carried out, it may be useful to cause a suction effect by the piston 6 with a further attempt at suction in the direction of the arrow f3 in Fig. 3, to check visually that no blood has penetrated into the syringe, as indicated by S in Fig. 4. It should be noted that the operations of suction of the liquid cause the displacement of the tubular component 18 moved by the piston 6 as shown in Fig. 2; the brief stroke in the direction of the arrow f2 to cause the expulsion of the air makes the piston 6 move slightly away from the tubular component 18 as shown at 20 in Fig. 3, and the component 18, not being moved in the direction of the arrow f2 by the rod 7 and certainly not being moved by the piston 6 which tends to move away from said element 18. The component 18, however, remains well within the conditions in which it surrounds the intermediate element 10. Any attempted suction in the direction f3 to check whether the needle is in a vein can slightly reduce the gap 20 between the tubular component 18 and the piston 6 as indicated by 20' in Fig. 4.

In any case, at the end of the suction, the expulsion of air and any check that the needle is not in a vein, as described above, the tubular component 18 remains in the condition of engagement of the rod 7 with the mushroom head 7A which is engaged with the intermediate element 10, owing to the presence of the tubular component 18 which remains in the condition of surrounding the element 10 and therefore of preventing the spreading of its end forming the housing 14.

When the injection of the liquid L is caused by action on the rod 7 of the piston 6 in the direction of the arrow f5 in Fig. 5, this causes the piston 6 to slide in the direction of expulsion of the liquid and thus also causes the excursion of the intermediate element 10, while the tubular component 18 continues to be retained by friction in the position which it reached previously; at the end of the injection stroke in the direction f5, the intermediate element 10 is completely released from the tubular component 18, and thus the configuration in Fig. 5 is obtained. In these conditions, after the needle has been withdrawn from the tissue T, if an attempt is made to retum the rod 7 in the direction of the arrow f6 in Fig. 6, the mushroom head 7A is disengaged from the housing 14 as a result of the spreading of the appendages delimited by the cuts 16, which can spread out as indicated by 16X in Figs. 6 and 7, so that the operation in the direction of the arrow f6 in Fig. 6 does not cause the return of the piston 6, which continues to be retained at the bottom of the barrel 5 of the syringe.

Thus the re-use of the described syringe is made impossible.

The drawing shows the solution in which the conventional components 6 and 7, 7A of a single-use syringe are used, and the intermediate element 10 and the tubular component 18 are additionally used. The invention can also be implemented by still using a tubular component 18, but by using a piston which includes an extension (in replacement of the combination of the piston 6 and the intermediate element 10 connected together), without a solution of continuity with the socket 6A and with the appendage 12, but using a single component for the function described previously of the two components 6 and 10 connected together, as shown in Fig. 10, in which a single component 100 forms the piston 102, the extension 104 and the expandable housing 106, said extension 104 being surrounded by the tubular component 18.

It is to be understood that the drawing shows only an example provided solely as a practical demonstration of the invention, and that this invention can be varied in its forms and arrangements without departure from the scope of the invention. The presence of any reference numbers in the attached claims has the purpose of facilitating the reading of the claims with reference to the description and to the drawing, and does not limit the scope of protection represented by the claims.

## Claims

1. Single-use syringe constructed to prevent its subsequent re-use, said syringe comprising a barrel (5) with a needle (1), a piston (6) made from elastomeric material and a piston rod (7), a disengageable connection with a socket (6A) and a mushroom head (7A) of the expansion type between the piston (6) and the rod (7), an intermediate element (10) able to be connected to the piston (6) and the rod (7), and a tubular component (18), which is capable of stabilizing the connection between said intermediate element (10) and the rod (7) is in frictional contact with the inner surface (5A) of the barrel (5) and capable of being moved by the piston (6) when the latter is made to slide in the direction (f1) of suction, the disengagement between the intermediate element (10) and the rod (7) being caused by the reverse stroke (f6) for injection, while the tubular component (18) continues to be retained by friction on said inner surface (5A) of the barrel (5), **characterized in that** said intermediate element (10) is provided with an appendage with a mushroom head (12) shaped in the same way as the mushroom head (7A) of the piston rod (7) and with a socket (14, 16) like the socket (6A) of the piston (6), said appendage (12) being engaged in the socket (6A) provided in the piston (6) to receive the mushroom head (7A) of the rod (7) so that the appendage replaces the latter mushroom head, and the rod (7, 7A) being engageable in said socket (14, 16).

2. Single-use syringe according to claim 1, **characterized in that** said tubular component (18) is a length of tube.

## Patentansprüche

1. Einwegspritze, die so aufgebaut ist, dass ihre erneute Verwendung verhindert wird, wobei die Spritze umfaßt: einen Hohlzylinder (5) mit einer Injektionskanüle (1), einen Kolben (6) aus elastomerem Werkstoff und eine Kolbenstange (7), eine lösbare Verbindung mit einer Muffe (6A) und ein konisch geformtes Kopfstück (7A) in der Art einer Spreizverbindung zwischen dem Kolben (6) und der Kolbenstange (7), ein Zwischenstück (10), das mit dem Kolben (6) und der Kolbenstange (7) verbunden werden kann und ein rohrförmiges Bauelement (18), das geeignet ist, die Verbindung zwischen dem besagten Zwischenstück (10) und der Kolbenstange (7) zu stabilisieren, das reibschlüssig Kontakt mit der Innenseite (5A) des Hohlzylinders (5) hat und durch den Kolben (6) bewegt werden kann, wenn letzterer in Richtung der Saugwirkung (f1) gleitend verschoben wird, wobei das Lösen des Zwischenstücks (10) von der Kolbenstange (7) bei der Injektion durch den Hub in die Gegenrichtung (f6) verursacht wird, während das rohrförmige Bauelement (18) infolge der Reibung an der Innenseite (5A) des Hohlzylinders (5) weiterhin festgehalten wird, **dadurch gekennzeichnet, dass** das Zwischenstück (10) mit einer Verlängerung, die ein konisch geformtes Kopfstück (12) aufweist, versehen ist, das genauso geformt ist wie das konisch geformte Kopfstück (7A) der Kolbenstange (7), und mit einer Muffe (14, 16), ähnlich der Muffe (6A) des Kolbens (6), wobei dieses konisch geformte Kopfstück (12) mit der Muffe (6A) im Eingriff ist, die im Kolben (6) vorgesehen ist, um das konisch geformte Kopfstück (7A) der Kolbenstange (7) aufzunehmen, so dass die Verlängerung an die Stelle des zuletzt genannten, konisch geformten Kopfstücks tritt und die Kolbenstange (7, 7A) mit der genannten Muffe (14, 16) in Eingriff gebracht werden kann.

2. Einwegspritze nach Anspruch 1, **dadurch gekennzeichnet, dass** das rohrförmige Bauelement (18) aus einem Rohrstück besteht.

## Revendications

1. Seringue jetable conçue pour empêcher sa réutilisation, ladite seringue comprenant un cylindre (5) avec une aiguille (1), un piston (6) constitué d'un élastomère et une tige de piston (7), une connexion séparable comprenant une cavité (6A) et une tête en forme de champignon (7A) du type à expansion entre le piston (6) et la tige (7), un élément intermédiaire (10) pouvant être connecté au piston (6) et à la tige (7), et un composant tubulaire (18), qui est capable de stabiliser la connexion entre ledit élément intermédiaire (10) et la tige (7) et qui est en contact frictionnel avec la surface interne (5A) du cylindre (5), ce composant pouvant être déplacé par le piston (6) lorsque ce dernier est amené à glisser dans la direction (f1) d'aspiration, le désaccouplement entre l'élément intermédiaire (10) et la tige (7) étant provoqué par la course de retour (f6) pour l'injection, tandis que le composant tubulaire (18) continue à être retenu par friction sur ladite surface interne (5A) du cylindre (5), **caractérisée en ce que** ledit élément intermédiaire (10) est doté d'un appendice avec une tête en forme de champignon (12) formée de la même manière que la tête en forme de champignon (7A) de la tige de piston (7) et avec une cavité (14, 16) similaire à la cavité (6A) du piston (6), ledit appendice (12) étant engagé dans la cavité (6A) ménagée dans le piston (6) pour recevoir la tête en forme de champignon (7A) de la tige (7), de manière à ce que l'appendice remplace ladite dernière tête en forme de champignon, et la tige (7, 7A) étant engageable dans ladite cavité (14, 16).

2. Seringue jetable selon la revendication 1, **caractérisée en ce que** ledit composant tubulaire (18) est constitué par une longueur de tube.
